# EUROPÄISCHE PATENTSCHRIFT

(11) **EP 3 417 838 B1**
(45) Veröffentlichungstag und Bekanntmachung des Hinweises auf die Patenterteilung: **15.03.2023**
(21) Anmeldenummer: 18179184.9
(22) Anmeldetag: 21.06.2018
(51) Int. Cl.: A61F 5/01

(54) **ELASTISCHES KORREKTURBAND UND SYSTEM ZUR KORREKTUR VON ZEHFEHLSTELLUNGEN EINES MENSCHLICHEN FUSSES**
ELASTIC CORRECTION TAPE AND SYSTEM FOR CORRECTION OF MALPOSITIONS OF A HUMAN FOOT
BANDE DE CORRECTION ÉLASTIQUE ET SYSTÈME DE CORRECTION DES MAUVAISES POSITIONS DES ORTEILS D'UN PIED HUMAIN

(30) Priorität: 22.06.2017 DE 202017103733 U
(43) Veröffentlichungstag der Anmeldung: 26.12.2018
(73) Patentinhaber: FXF GmbH, 92224 Amberg (DE)
(72) Erfinder: FISCHER, Franz, 92224 Amberg (DE)
(74) Vertreter: Benninger, Johannes

(56) Entgegenhaltungen:
- WO-A1-2012/077112
- DE-C- 838 481
- DE-U1- 9 204 651
- US-A- 1 899 092
- US-A- 3 556 091
- US-A- 4 632 103
- US-A1- 2014 207 042

## Beschreibung

Die vorliegende Erfindung betrifft ein elastisches Korrekturband mit den Merkmalen des unabhängigen Anspruchs 1. Die Erfindung betrifft zudem ein System zur Korrektur von Zehfehlstellungen eines menschlichen Fußes mit den Merkmalen des unabhängigen Anspruchs 5.

Fußfehlstellungen, insbesondere Hallux Valgus, treten krankheits- und/oder altersbedingt und/oder durch länger andauerndes Tragen einer falschen oder ungeeigneten Fußbekleidung bei größeren Teilen der Bevölkerung regelmäßig auf. Bei Hallux Valgus verändert die Großzehe ihre Position und wandert, ausgelöst durch eine Abflachung der inneren Längswölbung am Fuß und einer Außenrotation im Unterschenkel, nach außen ab. Je nach Ausprägung kann Hallux Valgus bei den davon betroffenen Personen zu Gehbinderungen und Schmerzen führen.

Hallux Valgus kann mittels Röntgenbild bildgebend und/oder mittels des Hallux Valguswinkels klinisch in verschiedene Ausprägungs- oder Schweregrade eingeteilt werden. Je nach Schweregrad stehen zur Therapie des Hallux Valgus konservative Verfahren, beispielsweise im Rahmen der Physiotherapie sowie der Orthopädietechnik, und/oder operative Verfahren zur Verfügung, wobei die Auswahl des Verfahrens zur Korrektur der Fehlstellung vom Schweregrad der Fehlstellung des Betroffenen abhängig ist. Zu den konservativen Verfahren gehören beispielsweise Schienen, Bandagen/Tapes, Socken mit eingearbeiteten Tapes und Silikonkeile.

Durch DE 201 12 537 U1 und DE 20 2005 006 701 U1 ist jeweils eine Großzehbandage offenbart, welche durch zumindest ein Elastikband gebildet ist. Die Großzehbandage umfasst zwei Verbindungen und einen Verschluss, wobei das Elastikband an zwei Kreuzungspunkten durch die beiden Verbindungen so zusammen gehalten wird, dass der Großzeh vom Elastikband derart umgeben ist, dass das Elastikband an der Oberseite des Großzehs entlang über den ersten Kreuzungspunkt am Ansatzpunkt des Großzehs am Fuß und auf der Unterseite des Fußes diagonal nach hinten zu einer Schlaufe um den Kreuzungspunkt herum geführt wird. Über den Verschluss kann die Bandage am Knöchel des Halters fixiert werden.

Die DE 92 04 651 U1 offenbart eine medizinische Orthese zur Behandlung von Belastungsdeformitäten im Bereich der Großzehe. Die Orthese besteht aus einem elastisch bandförmig ausgebildeten Material zur Korrektur von Hallux Valgus. Am vorderen Ende des Bandes ist ein circulär an der Großzehe angebrachter, in seinem Umfang stufenlos einstellbarer Klettverschluss fest befestigt. Mittig an der Außenseite des Bandes ist eine Kletthaftfläche vorgesehen, an der ein Klettband mit einem Ende befestigt ist, wobei das freie um den Mittelfuß legbare Ende des Klettbandes an der Kletthaftfläche befestigt ist. Zudem ist am hinteren Ende des Bandes, innenseitig ein Klettband befestigt, welches um den Knöchel gelegt und an der Kletthaftfläche befestigt ist.

Bei diesen Lösungen hat es sich jedoch als nachteilig erwiesen, dass die Bandage bei der Anwendung jeweils vom Träger selbst zu wickeln ist und/oder der Wickelvorgang sich als kompliziert erwiesen hat, da die Kreuzungspunkte jeweils an exakt vorgegebenen Stellen anzuordnen sind, um entsprechende Zugkräfte auf die Großzehe bewirken zu können. Bei mangelnder Erfahrung und bei fehlerhafter Wicklung kann folglich das Gegenteil einer Korrektur von Zehfehlstellungen bewirkt werden.

Eine weitere häufige Fußerkrankung stellt die Hammerzehe dar. Bei der Hammerzehe handelt es sich um eine permanente krallenartige Beugung der Zehe. Es kann zwischen einer flexiblen und einer fixierten Hammerzehe unterschieden werden. Ebenso wie beim Krankheitsbild des Hallux Valgus kann auch die Hammerzehe krankheits- und/oder altersbedingt auftreten und/oder durch eine falsche Fußbekleidung gefördert werden.

Durch die GB 445 921 A ist ein Mittel zur Korrektur von Fußfehlstellungen, insbesondere von Zehengelenken, wie beispielsweise bei einer Hammerzehe, offenbart. Auf einer Plattform kann eine Schlaufe vorgesehen sein, in welche Schlaufe die fehlgebildete Zehe eingeführt werden kann. Die Größe der Schlaufe und damit die auf den Zeh wirkende Kraft kann mittels einer Schnalle eingestellt werden. Diese Lösung hat sich jedoch insbesondere dahingehend als nachteilig erwiesen, dass die Schnalle je nach Position für den Betroffenen beim Tragen im Schuh sehr unangenehm ist, da es sehr leicht zu Druckstellen oder Unbequemlichkeiten kommen kann.

Angesichts der festgestellten Einschränkungen der bekannten Korrektursysteme liegt der vorliegenden Erfindung die Aufgabe zugrunde, ein Band oder Korrektursystem unter Verwendung eines elastischen Korrekturbandes zur Verfügung zu stellen, bei welchem die im Stand der Technik bekannten Nachteile zumindest teilweise überwunden werden können. Das System bzw. das elastische Korrekturband kann zugleich für eine prophylaktische Versorgung von Fuß- und/oder Zehfehlstellungen, insbesondere bei Hallux Valgus, sowie für eine postoperative Therapie zur Sicherung des Operationsergebnisses dienen. Weiter können mit dem elastischen Korrekturband der Fuß, insbesondere dessen Bänder und Gelenke, nach einem operativen Eingriff stabilisiert werden.

Die genannten Aufgaben werden gelöst durch ein elastisches Korrekturband mit den Merkmalen des unabhängigen Anspruchs 1 sowie durch ein System mit den Merkmalen des unabhängigen Anspruchs 5. Weitere vorteilhafte Ausgestaltungen werden durch die jeweils den unabhängigen Ansprüchen zugeordneten abhängigen Ansprüche beschrieben.

Das die Erfindung bildende elastische Korrekturband für Zehfehlstellungen, insbesondere für Hallux Valgus oder Hammerzehen, umfasst zumindest einen ersten Abschnitt, welcher um zumindest eine Zehe eines Fußes führbar ist und eine Schlaufe ausbildet. Bei der Schlaufe kann es sich um eine einseitig ausgebildete Schlaufe oder um eine offene Schlaufe handeln. Bei der zumindest einen Zehe kann es sich um die Großzehe und/oder um eine der kleinen Zehen des Fußes handeln. Die Größe der Schlaufe kann variierbar eingestellt werden und kann jeweils unterschiedlich groß ausgebildet sein. Vorzugsweise kann die Größe der Schlaufe individuell an die zumindest eine Zehe angepasst sein. Über die Schlaufe kann auch die Zugkraft des elastischen Korrekturbandes für die zumindest eine Zehe eingestellt werden.

Weiter umfasst das erfindungsgemäße elastische Korrekturband einen sich an die Schlaufe anschließenden wenigstens einen zweiten Abschnitt, welcher sich längsgerichtet zumindest abschnittsweise medial entlang einer Fußlängsseite bis zur Ferse erstreckt.

Wahlweise können sich an die Schlaufe auch wenigstens zwei zweite Abschnitte anschließen, welche beispielsweise medial und/oder dorsal an einer Fußlängsseite oder an einer Fußsohle des Fußes angeordnet sein können. Der wenigstens eine zweite Abschnitt kann daher wahlweise durch zwei Bandstreifen ausgebildet sein.

Zudem umfasst das elastische Korrekturband zumindest einen dritten Abschnitt, welcher um die Ferse führbar ist, wobei der zumindest eine dritte Abschnitt am sich an die Schlaufe anschließenden wenigstens einen zweiten Abschnitt des elastischen Korrekturbandes gegengelagert ist.

Der zumindest eine dritte Abschnitt ist innerhalb eines bestimmten Winkels zum zweiten Abschnitt geführt und/oder angeordnet, welcher Winkel zwischen 10 und 170 Grad, vorzugsweise zwischen 30 und 70 Grad, ausgebildet ist. Über den Grad des Winkels kann zusätzlich auch die durch das elastische Korrekturband erzeugte Zugkraft für die zumindest eine Zehe eingestellt werden.

Erfindungsgemäß ist der zumindest eine dritte Abschnitt im Bereich der Ferse am elastischen Korrekturband gegengelagert bzw. befestigt.

Der zumindest eine erste Abschnitt kann zumindest abschnittsweise in zumindest zwei gleichbreite elastische Korrekturbandstreifen aufgeteilt werden und/oder der zumindest eine erste Abschnitt kann an mindestens einer bestimmten Position eine Ausnehmung besitzen. Über die zumindest zwei gleichbreiten elastischen Korrekturbandstreifen und/oder über die mindestens eine Ausnehmung kann die jeweilige Zugkraft am zumindest einem Zeh zusätzlich dosiert werden.

Die mindestens eine Ausnehmung kann schlitzförmig und/oder in sonstiger Weise ausgestaltet sein. Vorzugsweise kann die mindestens eine Ausnehmung entlang der Längserstreckungsrichtung des elastischen Korrekturbandes angeordnet sein. Wahlweise kann die mindestens eine Ausnehmung auch quer zur Längserstreckungsrichtung des elastischen Korrekturbandes ausgebildet sein.

Gemäß einem Ausführungsbeispiel der Erfindung kann der sich an die Schlaufe anschließende wenigstens eine zweite Abschnitt zumindest abschnittsweise medial oder dorsal an der Fußlängsseite angeordnet sein. Der sich an die Schlaufe anschließende wenigstens eine zweite Abschnitt kann sich daher zumindest abschnittsweise entlang der Fußinnenseite oder der Fußaußenseite des Fußes erstrecken.

Gemäß einem nicht zur Erfindung gehöhrenden Ausführungsbeispiel kann sich der an die Schlaufe anschließende wenigstens eine zweite Abschnitt zumindest abschnittsweise über eine Fußsohle des Fußes erstrecken. Der sich an die Schlaufe anschließende wenigstens eine zweite Abschnitt kann sich zumindest weitgehend schräg orientiert und/oder diagonal über die Fußsohle des Fußes erstrecken.

Der sich an die Schlaufe anschließende wenigstens eine zweite Abschnitt und/oder der zumindest eine dritte Abschnitt können wenigstens einen Kreuzungspunkt ausbilden, indem der sich an die Schlaufe anschließende wenigstens eine zweite Abschnitt und/oder der zumindest eine dritte Abschnitt jeweils zumindest weitgehend schräg orientiert und/oder diagonal zueinander entlang einer Fußlängsseite erstrecken. Der Kreuzungspunkt kann beispielsweise an der Fußsohle und/oder an der Fußinnen- und/oder Fußaußenseite gebildet werden.

Der wenigstens eine Kreuzungspunkt kann zumindest weitgehend im vorderen Bereich der Fußsohle angeordnet sein, so dass eine zumindest vertikale Kraft nach unten ausgebildet wird. Wahlweise kann der wenigstens eine Kreuzungspunkt auch etwa mittig von der Fußsohle und/oder an jeglichen anderen Stelle entlang der Fußsohle angeordnet sein.

Wahlweise kann der wenigstens eine Kreuzungspunkt an einer beliebigen Position im Bereich der Fußinnen- und/oder Fußaußenseite vorgesehen sein.

Zudem können sich der zumindest eine erste Abschnitt und/oder der zumindest eine dritte Abschnitt jeweils mit dem elastischen Korrekturband ineinander übergehen. Wahlweise können sich der zumindest eine erste Abschnitt und/oder der zumindest eine dritte Abschnitt kreuzen.

Es kann vorgesehen sein, dass das Korrekturband in Richtung des zumindest einen ersten Abschnittes und/oder des zumindest einen dritten Abschnittes und/oder zumindest abschnittsweise an einer beliebigen Position verjüngt ausgebildet ist/sind.

Es kann vorgesehen sein, dass der zumindest eine erste Abschnitt und/oder der zumindest eine dritte Abschnitt jeweils am elastischen Korrekturband stoff- und/oder kraftschlüssig und/oder in sonstiger Weise am elastischen Korrekturband befestigt ist. Vorzugsweise sind jeweils der zumindest erste Abschnitt und/oder der zumindest eine dritte Abschnitt mit dem elastischen Korrekturband miteinander verklebt und/oder verschweißt.

Das elastische Korrekturband kann durch ein flexibles und/oder gummiartiges Material ausgebildet sein.

Neben dem zuvor in verschiedenen Ausführungsvarianten beschriebenen elastischen Korrekturband, das für Zehfehlstellungen, insbesondere für Hallux Valgus vorgesehen ist, bezieht sich die vorliegenden Erfindung auch auf ein System zur Korrektur von Zehfehlstellungen eines menschlichen Fußes unter Verwendung eines am Fuß befestigten und mit den zu korrigierenden Zehen wechselwirkenden elastischen Korrekturbandes, das insbesondere gemäß einer der zuvor beschriebenen Ausführungsvarianten ausgebildet sein kann. Das für das erfindungsgemäße System eingesetzte elastische Korrekturband umfasst zumindest einen ersten Abschnitt, welcher um zumindest eine Zehe eines Fußes führbar ist und eine Schlaufe ausbildet. Bei der Schlaufe kann es sich um eine einseitig ausgebildete Schlaufe oder um eine offene Schlaufe handeln. Bei der zumindest einen Zehe kann es sich um die Großzehe und/oder um eine der kleinen Zehen des Fußes handeln. Die Größe der Schlaufe kann variierbar eingestellt werden und kann jeweils unterschiedlich groß ausgebildet sein. Vorzugsweise kann die Größe der Schlaufe individuell an die zumindest eine Zehe angepasst sein. Über die Schlaufe kann auch die Zugkraft des elastischen Korrekturbandes für die zumindest eine Zehe eingestellt werden.

Weiter umfasst das elastische Korrekturband einen sich an die Schlaufe anschließenden wenigstens einen zweiten Abschnitt, welcher sich längsgerichtet zumindest abschnittsweise entlang einer Fußlängsseite bis zur Ferse erstreckt. Der wenigstens sich an die Schlaufe anschließende zweite Abschnitt kann sich zumindest abschnittsweise medial und/oder dorsal entlang einer Fußlängsseite oder entlang einer Fußsohle des Fußes erstrecken.

Wahlweise können sich an die Schlaufe auch wenigstens zwei zweite Abschnitte anschließen, welche beispielsweise medial und/oder dorsal an einer Fußlängsseite oder an einer Fußsohle des Fußes angeordnet sein können. Der wenigstens eine zweite Abschnitt kann daher wahlweise durch zwei Bandstreifen ausgebildet sein.

Zudem umfasst das elastische Korrekturband zumindest einen dritten Abschnitt, welcher um die Ferse führbar ist, wobei der zumindest eine dritte Abschnitt am sich an die Schlaufe anschließenden wenigstens einen zweiten Abschnitt des elastischen Korrekturbandes gegengelagert ist oder um die zumindest eine Zehe oder um eine weitere Zehe führbar ist, indem der zumindest eine dritte Abschnitt eine weitere Schlaufe ausbildet.

Aus dem Gesagten wird deutlich, dass das Korrekturband beim Tragen in Wechselwirkung mit dem menschlichen Fuß des Trägers tritt, indem es Zugkräfte in bestimmte Richtungen auf die Zehen aufprägt, die von den Schlaufen des Korrekturbandes umschlungen sind. Das Band kann in vorteilhafter Weise an der Fußinnenseite entlang der Längsrichtung des Fußes geführt sein, oberhalb der Ferse, insbesondere im Bereich der Achillessehne, um den Fuß herumgeführt und entlang der Fußsohle wieder in Richtung zu den Zehen verlaufen und entweder dort in Querrichtung oder einem bestimmten Winkel am Band selbst verankert sein, bspw. auf halber Länge des Fußes, oder in einer weiteren Schlaufe unter Ausbildung eines Gegenlagers an einer weiteren Zehe oder ggf. auch an der zu korrigierenden Zehe mittels der weiteren Schlaufe verankert sein. Auf diese Weise ist das Band gespannt und übt die gewünschten Zugkräfte in die gewünschte Richtung auf den umschlungenen Zeh aus und ist gleichzeitig aufgrund der gleichmäßigen Zugkraftverteilung über die gesamte Länge des Bandes und dessen Verlauf über den Fuß komfortabel zu tragen, so dass keine zu starken Zugkräfte oder Druckstellen o. dgl. entstehen, die auf Dauer zu Einschränkungen, zu Komfortverlusten und letztlich zu mangelnder Akzeptanz des Korrektursystems beim Träger führen könnten.

Der zumindest eine dritte Abschnitt kann innerhalb eines bestimmten Winkels zum zweiten Abschnitt geführt und/oder angeordnet sein, welcher Winkel zwischen 10 und 170 Grad, vorzugsweise zwischen 30 und 70 Grad, ausgebildet sein kann. Über den Grad des Winkels kann zusätzlich auch die durch das elastische Korrekturband erzeugte Zugkraft für die zumindest eine Zehe eingestellt werden.

Wahlweise kann der zumindest eine dritte Abschnitt im Bereich der Ferse am elastischen Korrekturband gegengelagert bzw. befestigt sein.

Der zumindest eine erste Abschnitt kann zumindest abschnittsweise in zumindest zwei gleichbreite elastische Korrekturbandstreifen aufgeteilt werden und/oder der zumindest eine erste Abschnitt kann an mindestens einer bestimmten Position eine Ausnehmung besitzen. Über die zumindest zwei gleichbreiten elastischen Korrekturbandstreifen und/oder über die mindestens eine Ausnehmung kann die jeweilige Zugkraft am zumindest einem Zeh zusätzlich dosiert werden.

Die mindestens eine Ausnehmung kann schlitzförmig und/oder in sonstiger Weise ausgestaltet sein. Vorzugsweise kann die mindestens eine Ausnehmung entlang der Längserstreckungsrichtung des elastischen Korrekturbandes angeordnet sein. Wahlweise kann die mindestens eine Ausnehmung auch quer zur Längserstreckungsrichtung des elastischen Korrekturbandes ausgebildet sein.

Gemäß einem Ausführungsbeispiel der Erfindung kann der sich an die Schlaufe anschließende wenigstens eine zweite Abschnitt zumindest abschnittsweise medial oder dorsal an der Fußlängsseite angeordnet sein. Der sich an die Schlaufe anschließende wenigstens eine zweite Abschnitt kann sich daher zumindest abschnittsweise entlang der Fußinnenseite oder der Fußaußenseite des Fußes erstrecken.

Gemäß einem nicht zur Erfindung gehörenden Ausführungsbeispiel kann sich der an die Schlaufe anschließende wenigstens eine zweite Abschnitt zumindest abschnittsweise über eine Fußsohle des Fußes erstrecken. Der sich an die Schlaufe anschließende wenigstens eine zweite Abschnitt kann sich zumindest weitgehend schräg orientiert und/oder diagonal über die Fußsohle des Fußes erstrecken.

Der sich an die die Schlaufe anschließende wenigstens eine zweite Abschnitt und/oder der zumindest eine dritte Abschnitt können wenigstens einen Kreuzungspunkt ausbilden, indem der sich an die Schlaufe anschließende wenigstens eine zweite Abschnitt und/oder der zumindest eine dritte Abschnitt jeweils zumindest weitgehend schräg orientiert und/oder diagonal zueinander entlang einer Fußlängsseite erstrecken. Der Kreuzungspunkt kann beispielsweise an der Fußsohle und/oder an der Fußinnen- und/oder Fußaußenseite gebildet werden.

Der wenigstens eine Kreuzungspunkt kann zumindest weitgehend im vorderen Bereich der Fußsohle angeordnet sein, so dass eine zumindest vertikale Kraft nach unten ausgebildet wird. Wahlweise kann der wenigstens eine Kreuzungspunkt auch etwa mittig von der Fußsohle und/oder an jeglichen anderen Stelle entlang der Fußsohle angeordnet sein.

Wahlweise kann der wenigstens eine Kreuzungspunkt an einer beliebigen Position im Bereich der Fußinnen- und/oder Fußaußenseite vorgesehen sein.

Zudem können sich der zumindest eine erste Abschnitt und/oder der zumindest eine dritte Abschnitt jeweils mit dem elastischen Korrekturband ineinander übergehen. Wahlweise können sich der zumindest eine erste Abschnitt und/oder der zumindest eine dritte Abschnitt kreuzen.

Es kann vorgesehen sein, dass das Korrekturband in Richtung des zumindest einen ersten Abschnittes und/oder des zumindest einen dritten Abschnittes und/oder zumindest abschnittsweise an einer beliebigen Position verjüngt ausgebildet ist/sind.

Es kann vorgesehen sein, dass der zumindest erste Abschnitt und/oder der zumindest eine dritte Abschnitt jeweils am elastischen Korrekturband stoff- und/oder kraftschlüssig und/oder in sonstiger Weise am elastischen Korrekturband befestigt ist. Vorzugsweise sind jeweils der zumindest erste Abschnitt und/oder der zumindest eine dritte Abschnitt mit dem elastischen Korrekturband miteinander verklebt und/oder verschweißt.

Das elastische Korrekturband, das bei dem erfindungsgemäßen System eingesetzt wird, kann insbesondere durch ein flexibles und/oder gummiartiges und/oder längselastisches Material ausgebildet sein.

Wie oben bereits mehrfach klargestellt, kann das erfindungsgemäße elastische Korrekturband insbesondere zur Behandlung des Krankheitsbildes "Hallux Valgus" eingesetzt werden und bildet beim bestimmungsgemäßen Tragen am menschlichen Fuß ein erfindungsgemäßes System zur Korrektur von derartigen Zehfehlstellungen. Das elastische Korrekturband umfasst wenigstens einen ersten Abschnitt, der um eine Zehe oder um mehrere Zehen geführt ist und hierbei eine einseitige Schlaufe ausbildet. Bei der Zehe kann es sich insbesondere um die Großzehe des Fußes handeln. An diese Schlaufe schließt sich in Längserstreckungsrichtung des Bandes wenigstens ein zweiter Abschnitt an, welcher sich längsgerichtet zumindest abschnittsweise entlang einer Fußlängsseite bis zur Ferse erstreckt. Der sich an die Schlaufe anschließende wenigstens eine zweite Abschnitt ist zumindest abschnittsweise medial am Fuß bzw. an einer Fußinnenseite des Fußes angeordnet.

Das elastische Korrekturband umfasst außerdem zumindest einen dritten Abschnitt, welcher um die Ferse des Fußes geführt ist und am sich an die Schlaufe anschließenden wenigstens einen zweiten Abschnitt des elastischen Korrekturbandes gegengelagert bzw. dort verankert bzw. fixiert ist. Zudem ist der zumindest eine dritte Abschnitt schräg orientiert über die Fußsohle des Fußes 32 führbar bzw. geführt, so dass der zumindest eine dritte Abschnitt in Bezug auf den sich an die Schlaufe anschließenden wenigstens einen zweiten Abschnitt typischerweise einen Winkel zwischen circa 10° und 170°, vorzugsweise zwischen 30 und 70°, einschließt. D.h. ein in der Praxis sinnvoller Winkel kann etwa 50° bzw. 45° ... 55° betragen. Über die Anstellung bzw. über den Winkel des zumindest einen dritten Abschnittes wird die jeweilige Zugkraft zur Korrektur des Hallux Valgus eingestellt.

Der zumindest eine erste Abschnitt und der zumindest eine dritte Abschnitt sind jeweils am elastischen Korrekturband stoff- und/oder kraftschlüssig verbunden. Vorzugsweise sind der zumindest eine erste Abschnitt und der zumindest eine dritte Abschnitt mit dem elastischen Korrekturband verklebt oder verschweißt, wahlweise auch vernäht oder auf andere Weise fixiert. Dabei gehen der zumindest eine erste Abschnitt und der zumindest eine dritte Abschnitt jeweils mit elastischen Korrekturband ineinander über oder kreuzen sich zumindest abschnittsweise.

Der zumindest eine erste Abschnitt des elastischen Korrekturbandes ist zumindest abschnittsweise in zumindest zwei gleichbreite elastische Korrekturbandstreifen aufgeteilt oder kann an mindestens einer bestimmten Position eine längliche oder schlitzartige Ausnehmung aufweisen. Die mindestens eine Ausnehmung ist schlitzförmig ausgebildet und erstreckt sich zumindest abschnittsweise entlang der Längserstreckungsrichtung des elastischen Korrekturbandes. Die mindestens eine Ausnehmung ist vorzugsweise in einem Bereich angeordnet, der an der Großzehe anliegt oder diese teilweise umschlingt. Durch die mindestens eine Ausnehmung kann die auf die Großzehe wirkende Zugkraft zusätzlich dosiert und eingestellt werden. Die auf die Großzehe wirkende Zugkraft wird durch das elastische Korrekturband derart ausgebildet und eingestellt, dass die Großzehe gegenüber den weiteren Zehen mit einer Zugkraft nach außen beaufschlagt wird, so dass die Großzehen in ihre natürliche Position wandert.

Das elastische Korrekturband kann in Richtung des zumindest eines ersten Abschnitts und des zumindest einen weiteren Abschnitts verjüngt ausgebildet sein.

Wahlweise kann das elastische Korrekturband, welches jedoch nicht zur Erfindung gehört, auch zur Korrektur von Hammerzehen eingesetzt werden. Hierbei umfasst das elastische Korrekturband wiederum zumindest einen ersten Abschnitt, welcher um zumindest eine Zehe geführt ist und eine Schlaufe ausbildet. Bei der zumindest einen Zehe kann es sich bspw. um die zweite Zehe des Fußes handeln. Die Schlaufe trägt dazu bei, dass die Zehe über das elastische Korrekturband mit einer definierten Zugkraft beaufschlagt wird, welche vertikal nach unten gerichtet ist. Hierbei ist die Schlaufe in Richtung der Fußsohle offen ausgebildet, so dass eine einseitig offen ausgebildete Schlaufe ausgebildet wird.

An die Schlaufe schließt sich wenigstens ein zweiter Abschnitt an, welcher durch zwei Bandstreifen ausgebildet sein kann. Der wenigstens eine zweite Abschnitt erstreckt sich längsgerichtet zumindest abschnittsweise entlang einer Fußlängsseite, vorzugsweise entlang der Fußsohle, bis zur Ferse. Die zwei Bandstreifen sind schräg orientiert bzw. diagonal zueinander über die Fußsohle des Fußes verlaufend angeordnet, so dass wenigstens ein Kreuzungspunkt ausgebildet wird. Der wenigstens eine Kreuzungspunkt ist zumindest im vorderen Bereich der Fußsohle vorgesehen bzw. angeordnet.

Der zumindest eine dritte Abschnitt des wenigstens einen elastischen Korrekturbandes ist um die Ferse des Fußes geführt bzw. führbar und ist im Bereich der Ferse am elastischen Korrekturband gegengelagert, wodurch ebenfalls die jeweilige Zugkraft des elastischen Korrekturbandes eingestellt werden kann. Grundsätzlich gilt hierbei: je stärker das elastische Korrekturband gespannt ist, desto größer ist die jeweilige Zugkraft. Umgekehrt gilt genauso: je geringer das elastische Korrekturband gespannt ist, desto kleiner ist die jeweilige Zugkraft.

Der zumindest eine erste Abschnitt und der zumindest eine dritte Abschnitt sind jeweils am elastischen Korrekturband stoff- und/oder kraftschlüssig verbunden. Vorzugsweise sind der zumindest eine erste Abschnitt und der zumindest eine dritte Abschnitt mit dem elastischen Korrekturband verklebt oder verschweißt, wahlweise auch vernäht, verklammert oder anderweitig fixiert. Dabei gehen der zumindest eine erste Abschnitt und der zumindest eine dritte Abschnitt jeweils mit dem elastischen Korrekturband ineinander über oder kreuzen sich zumindest abschnittsweise.

Durch das elastische Korrekturband wird im Bereich der Schlaufe eine zumindest weitgehend vertikal nach unten gerichtete Zugkraft ausgebildet, so dass die Zehe bzw. die Hammerzehe in ihre natürliche Position gebracht wird.

Darüber hinaus kann das elastische Korrekturband im Bereich des zumindest einen ersten Abschnitts verjüngt ausgebildet sein, wobei der erste Abschnitt um die zumindest eine Zehe geführt ist.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.

Figuren 1A bis 1C zeigen ein Ausführungsbeispiel des elastischen Korrekturbandes am Fuß in unterschiedlichen Perspektiven.

Figuren 2A bis 2C zeigen ein weiteres, jedoch nicht zur Erfindung gehörendes, Ausführungsbeispiel eines elastischen Korrekturbands am Fuß in unterschiedlichen Perspektiven.

Figuren 3A und 3B zeigen ein weiteres, jedoch nicht zur Erfindung gehörendes, Ausführungsbeispiel eines elastischen Korrekturbands am Fuß in unterschiedlichen Perspektiven.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie die das elastische Korrekturband ausgestaltet sein kann und stellen keine abschließende Begrenzung dar.

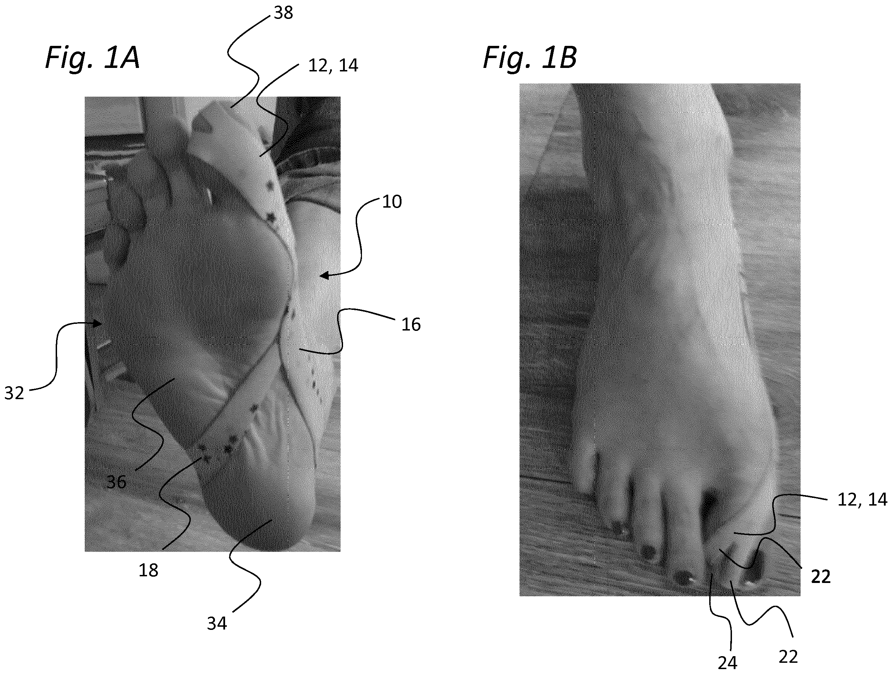

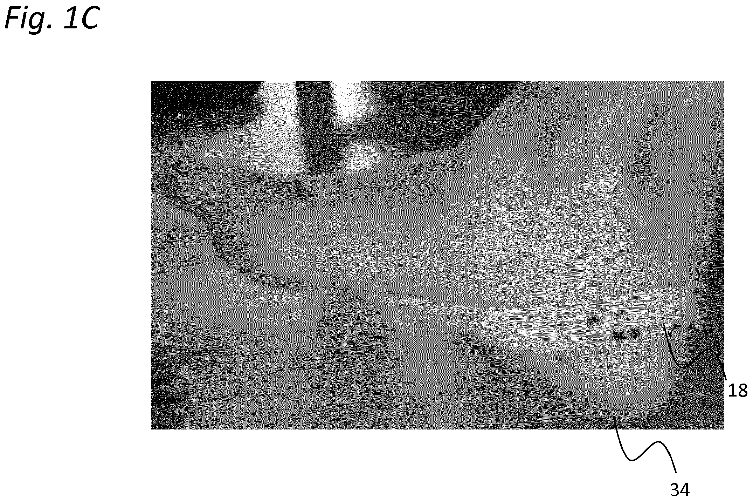

Die Figuren 1A bis 1C zeigen ein Ausführungsbeispiel des elastischen Korrekturbandes 10, welches insbesondere zur Behandlung von Hallux Valgus eingesetzt wird und beim bestimmungsgemäßen Tragen am menschlichen Fuß ein erfindungsgemäßes System zur Korrektur von Zehfehlstellungen bildet. Das elastische Korrekturband 10 umfasst zumindest einen ersten Abschnitt 12, welcher um zumindest eine Zehe 28 geführt ist und eine einseitige Schlaufe 14 ausbildet. Bei der zumindest einen Zehe 28 handelt es sich bei den Figuren 1A bis 1C um die Großzehe 38 des Fußes 32.

An die Schlaufe 14 schließt sich wenigstens ein zweiter Abschnitt 16 an, welcher sich längsgerichtet zumindest abschnittsweise entlang einer Fußlängsseite bis zur Ferse 34 erstreckt. Der sich an die Schlaufe 14 anschließende wenigstens eine zweite Abschnitt 16 ist zumindest abschnittsweise medial am Fuß 32 bzw. an einer Fußinnenseite des Fußes 32 angeordnet.

Das elastische Korrekturband 10 umfasst zumindest einen dritten Abschnitt 18, welcher um die Ferse 34 des Fußes 32 geführt ist und am sich an die Schlaufe 12 anschließenden wenigstens einen zweiten Abschnitt 16 des elastischen Korrekturbandes 10 gegengelagert ist. Zudem ist der zumindest eine dritte Abschnitt 18 schräg orientiert über die Fußsohle 36 des Fußes 32 führbar, so dass der zumindest eine dritte Abschnitt 18 in Bezug auf den sich an die Schlaufe 14 anschließenden wenigstens einen zweiten Abschnitt 16 vorzugsweise einen Winkel zwischen circa 10° und 170°, vorzugsweise zwischen 30 und 70°, einschließt. Über die Anstellung bzw. über den Winkel des zumindest einen dritten Abschnittes 19 wird die jeweilige Zugkraft zur Korrektur des Hallux Valgus eingestellt.

Der zumindest eine erste Abschnitt 12 und der zumindest eine dritte Abschnitt 18 sind jeweils am elastischen Korrekturband 10 stoff- und/oder kraftschlüssig verbunden. Vorzugsweise sind der zumindest eine erste Abschnitt 12 und der zumindest eine dritte Abschnitt 18 mit dem elastischen Korrekturband 10 verklebt oder verschweißt. Dabei gehen der zumindest eine erste Abschnitt 12 und der zumindest eine dritte Abschnitt 18 jeweils mit elastischen Korrekturband 10 ineinander über oder kreuzen sich zumindest abschnittsweise.

Der zumindest eine erste Abschnitt 12 des elastischen Korrekturbandes 10 ist zumindest abschnittsweise in zumindest zwei gleichbreite elastische Korrekturbandstreifen 22 aufgeteilt bzw. besitzt an mindestens einer bestimmten Position eine Ausnehmung 24. Die mindestens eine Ausnehmung 24 ist schlitzförmig ausgebildet und erstreckt sich zumindest abschnittsweise entlang der Längserstreckungsrichtung des elastischen Korrekturbandes 10. Die mindestens eine Ausnehmung 24 ist vorzugsweise im Bereich der Großzehe 38 angeordnet. Durch die mindestens eine Ausnehmung 24 kann die auf die Großzehe 38 wirkende Zugkraft zusätzlich dosiert und eingestellt werden.

Die Zugkraft wird durch das elastische Korrekturband 10 derart ausgebildet und eingestellt, dass die Großzehe gegenüber den weiteren Zehen 30 mit einer Zugkraft nach außen beaufschlagt wird, so dass die Großzehen in ihre natürliche Position wandert.

Das elastische Korrekturband 10 ist in Richtung des zumindest eines ersten Abschnitts 12 und des zumindest einen weiteren Abschnitts 18 verjüngt ausgebildet.

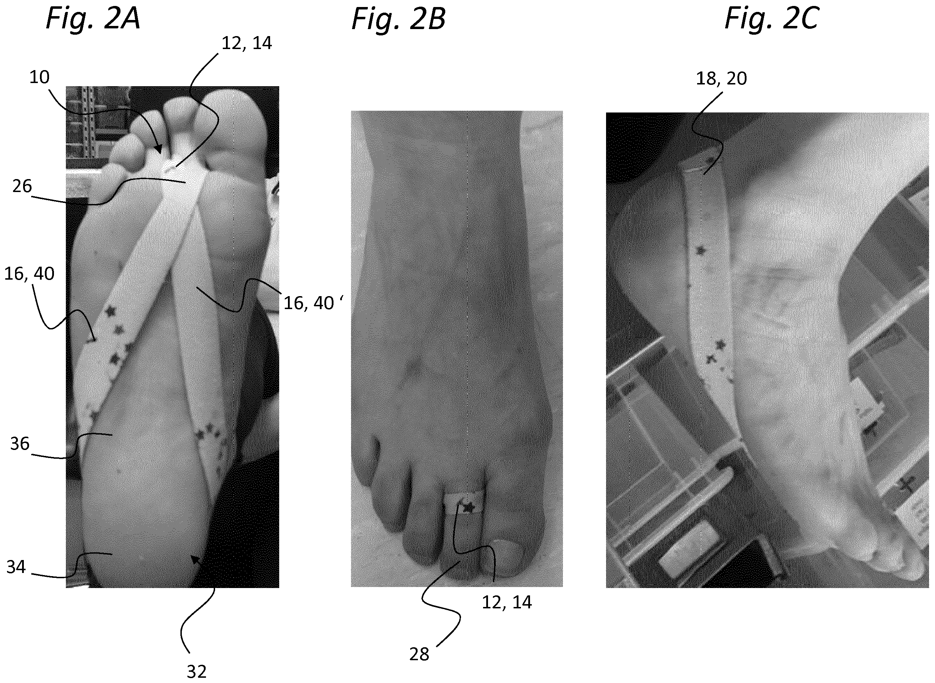

Die Figuren 2A bis 2C zeigen ein weiteres, jedoch nicht zur Erfindung gehörendes, Ausführungsbeispiel eines elastischen Korrekturbandes 10, welches insbesondere zur Korrektur von Hammerzehen eingesetzt wird. Das elastische Korrekturband 10 umfasst zumindest einen ersten Abschnitt 12, welcher um zumindest eine Zehe 28 geführt ist und eine Schlaufe 14 ausbildet. Bei der zumindest einen Zehe 28 handelt es sich um die zweite Zehe 40 des Fußes 32. Die Schlaufe 14 trägt dazu bei, dass die Zehe 28 über das elastische Korrekturband 10 mit einer definierten Zugkraft beaufschlagt wird, welche in ungefähr vertikaler Richtung nach unten gerichtet ist. Hierbei ist die Schlaufe 14 in Richtung der Fußsohle 32 offen ausgebildet, so dass eine einseitig offen ausgebildete Schlaufe 14 ausgebildet wird.

An die Schlaufe 14 schließt sich wenigstens ein zweiter Abschnitt 16 an, welcher durch zwei Bandstreifen 40, 40` ausgebildet ist. Der wenigstens eine zweite Abschnitt 16 erstreckt sich längsgerichtet zumindest abschnittsweise entlang einer Fußlängsseite, vorzugsweise entlang der Fußsohle 36, bis zur Ferse 34. Die zwei Bandstreifen 40, 40` sind schräg orientiert bzw. diagonal zueinander über die Fußsohle 36 des Fußes 32 angeordnet, so dass wenigstens ein Kreuzungspunkt 26 ausgebildet wird. Der wenigstens eine Kreuzungspunkt 26 ist zumindest im vorderen Bereich der Fußsohle 36 vorgesehen.

Der zumindest eine dritte Abschnitt 18 des wenigstens einen elastischen Korrekturbandes 10 ist um die Ferse 34 des Fußes 32 führbar und ist im Bereich der Ferse 32 am elastischen Korrekturband 10 gegengelagert, wodurch ebenfalls die jeweilige Zugkraft des elastischen Korrekturbandes 10 eingestellt werden kann. Grundsätzlich gilt: je stärker das elastische Korrekturband 10 gespannt ist, desto größer ist die jeweilige Zugkraft. Umgekehrt gilt genauso: je geringer das elastische Korrekturband gespannt ist, desto kleiner ist die jeweilige Zugkraft.

Der zumindest eine erste Abschnitt 12 und der zumindest eine dritte Abschnitt 18 sind jeweils am elastischen Korrekturband 10 stoff- und/oder kraftschlüssig verbunden. Vorzugsweise sind der zumindest eine erste Abschnitt 12 und der zumindest eine dritte Abschnitt 18 mit dem elastischen Korrekturband 10 verklebt oder verschweißt. Dabei gehen der zumindest eine erste Abschnitt 12 und der zumindest eine dritte Abschnitt 18 jeweils mit elastischen Korrekturband 10 ineinander über oder kreuzen sich zumindest abschnittsweise.

Durch das elastische Korrekturband 10 wird im Bereich der Schlaufe 14 eine zumindest weitgehend vertikal nach unten gerichtete Zugkraft ausgebildet, so dass die Zehe 28 bzw. die Hammerzehe in ihre natürliche Position gebracht wird.

Darüber hinaus ist das elastische Korrekturband 10 vorzugsweise im Bereich des zumindest einen ersten Abschnitts 12 verjüngt ausgebildet, welcher zumindest eine erste Abschnitt um die zumindest eine Zehe 28 geführt ist.

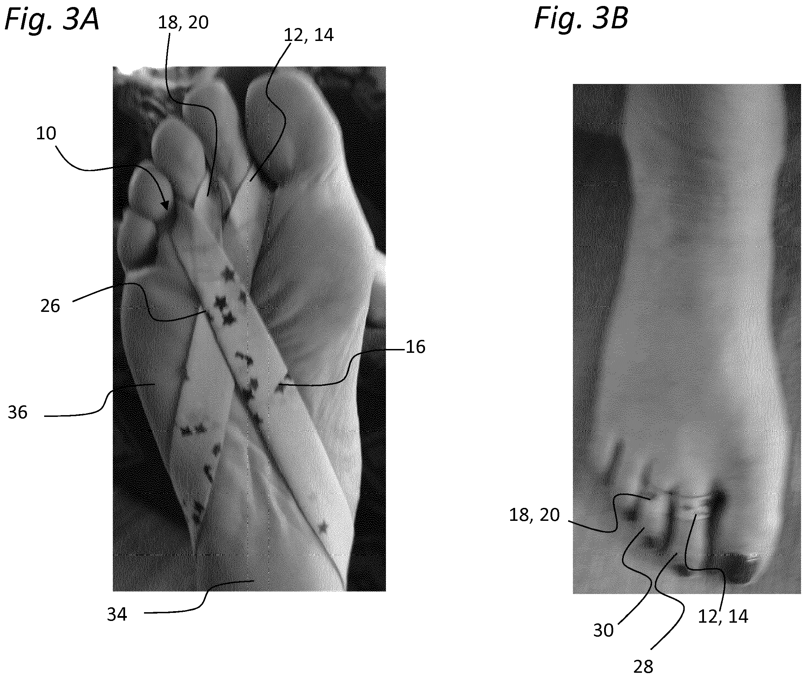

Die Figuren 3A und 3B zeigen ein weiteres, jedoch nicht zur Erfindung gehörendes, Ausführungsbeispiel eines elastischen Korrekturbandes 10, welches insbesondere zur Korrektur von Hammerzehen eingesetzt wird. Das elastische Korrekturband 10 umfasst zumindest einen ersten Abschnitt 12, welches um zumindest eine Zehe 28 geführt ist und eine einseitige Schlaufe 14 ausbildet. Bei der zumindest einen Zehe 28 handelt es sich um die zweite Zehe 40 des Fußes 32. Die Schlaufe 14 trägt dazu bei, dass die Zehe 28 über das elastische Korrekturband 10 mit einer definierten Zugkraft beaufschlagt wird, welche nach vertikal nach unten gerichtet ist.

An die Schlaufe 14 schließt sich wenigstens ein zweiter Abschnitt 16 an, welcher sich längsgerichtet zumindest abschnittsweise entlang einer Fußlängsseite bis zur Ferse 34 erstreckt. Vorzugsweise erstreckt sich der sich an die Schlaufe 14 anschließende wenigstens eine zweite Abschnitt 16 über die Fußsohle 36 des Fußes.

Das elastische Korrekturband 10 umfasst weiter zumindest einen dritten Abschnitt 18, welcher um die Ferse 34 sowie um eine weitere Zehe 30 des Fußes 32 geführt ist, indem der zumindest eine dritte Abschnitt 18 eine weitere Schlaufe 20 ausbildet.

Der sich an die Schlaufe 14 anschließende wenigstens eine zweite Abschnitt 16 und der zumindest eine dritte Abschnitt 18 verlaufen auf der Fußsohle 36 des Fußes 32 zumindest abschnittweise schräg orientiert bzw. zumindest diagonal zueinander und bilden im vorderen Bereich der Fußsohle 26 wenigstens einen Kreuzungspunkt 26 aus. Vorzugsweise ist der wenigstens eine Kreuzungspunkt zwischen der zumindest einen Zehe 28 und der zumindest einen weiteren Zehe 30 des Fußes 32 angeordnet.

Der zumindest eine erste Abschnitt 12 und der zumindest eine dritte Abschnitt 18 sind jeweils am elastischen Korrekturband 10 stoff- und/oder kraftschlüssig verbunden. Vorzugsweise sind der zumindest eine erste Abschnitt 12 und der zumindest eine dritte Abschnitt 18 mit dem elastischen Korrekturband 10 verklebt oder verschweißt. Dabei gehen der zumindest eine erste Abschnitt 12 und der zumindest eine dritte Abschnitt 18 jeweils mit elastischen Korrekturband 10 ineinander über oder kreuzen sich zumindest abschnittsweise.

Durch das elastische Korrekturband 10 wird im Bereich der Schlaufe 14 eine zumindest weitgehend vertikal nach unten gerichtete Zugkraft ausgebildet, so dass sowohl die Zehe 28 und die weitere Zehe 30 in ihre natürliche Position gebracht wird.

Darüber hinaus ist das elastische Korrekturband 10 in Richtung des zumindest einen ersten Abschnittes 12 und/oder des zumindest einen dritten Abschnittes 18 verjüngt ausgebildet.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

- 10: elastisches Korrekturband
- 12: erster Abschnitt
- 14: Schlaufe
- 16: zweiter Abschnitt
- 18: dritter Abschnitt
- 20: weitere Schlaufe
- 22: Korrekturbandstreifen
- 24: Ausnehmung
- 26: Kreuzungspunkt
- 28: Zehe
- 30: weitere Zehe
- 32: Fuß
- 34: Ferse
- 36: Fußsohle
- 38: Großzehe
- 40: Bandstreifen
- 40': Bandstreifen

## Patentansprüche

1. Elastisches Korrekturband (10) für bzw. zum Einsatz bei Zehfehlstellungen, aufweisend:
- zumindest einen ersten Abschnitt (12), welcher im Gebrauch um zumindest eine Zehe (28) eines Fußes (32) geführt ist und eine Schlaufe (14) ausbildet,
- wenigstens einen sich an die Schlaufe (14) anschließenden zweiten Abschnitt (16), welcher im Gebrauch sich längsgerichtet zumindest abschnittsweise medial entlang einer Fußlängsseite bis zur Ferse (34) erstreckt,
- zumindest einen dritten Abschnitt (18), welcher im Gebrauch um die Ferse (34) des Fußes (32) geführt ist,
**dadurch gekennzeichnet, dass** der zumindest eine dritte Abschnitt (18) am sich an die Schlaufe (14) anschließenden zweiten Abschnitt (16) des elastischen Korrekturbandes (10) gegengelagert ist,
und dass der zumindest eine dritte Abschnitt (18) im Gebrauch schräg orientiert über die Fußsohle des Fußes (32) führbar bzw. geführt ist, so dass der zumindest eine dritte Abschnitt (18) in Bezug auf den sich an die Schlaufe (14) anschließenden wenigstens einen zweiten Abschnitt (16) typischerweise einen Winkel zwischen circa 10° und 170°, vorzugsweise zwischen 30 und 70°, einschließt.

2. Elastisches Korrekturband nach Anspruch 1, bei welchem der zumindest eine erste Abschnitt (12) zumindest abschnittsweise in seiner Längserstreckungsrichtung in etwa zwei gleichbreite elastische Korrekturbandstreifen (22) aufgeteilt ist, oder bei welchem der zumindest eine erste Abschnitt (18) an mindestens einer bestimmten Position eine schlitzartige Ausnehmung (24) besitzt.

3. Elastisches Korrekturband nach Anspruch 1 oder 2, bei welchem der zumindest eine erste Abschnitt (12) und/oder der zumindest eine dritte Abschnitt (18) jeweils mit dem Korrekturband (10) ineinander übergehen.

4. Elastisches Korrekturband nach einem der vorherigen Ansprüche, bei welchem der zumindest eine erste Abschnitt (12) und/oder der zumindest eine dritte Abschnitt (18) jeweils am elastischen Korrekturband (10) stoff- und/oder kraftschlüssig und/oder in sonstiger Weise am elastischen Korrekturband (10) befestigt ist.

5. System für bzw. zum Einsatz bei Zehfehlstellungen eines menschlichen Fußes, welches ein elastisches Korrekturband (10) gemäß einem der Ansprüche 1 bis 4 umfasst.

## Claims

1. An elastic correction strap (10) for use in the case of toe malpositions, the correction strap (10) having:
- at least one first section (12), which is guided around at least one toe (28) of a foot (32) and forms a loop (14) when in use,
- at least one second section (16) attaching to the loop (14), which second section (16) extends lengthwise at least in sections medially along a longitudinal foot side to the heel (34) when in use,
- at least one third section (18), which is guided around the heel (34) of the foot (32) when in use,
**characterised in that** the at least one third section (18) is counter supported at the second section (16) of the elastic correction strap (10), which second section attaches to the loop (14),
and **in that** the at least one third section (18) is guidable or guided over the sole of the foot (32) in an oblique orientation when in use, such that the at least one third section (18) typically encloses an angle between approximately 10° and 170°, preferably between 30° and 70°, in relation to the at least one second section (16) attaching to the loop (14).

2. The elastic correction strap according to claim 1, in which the at least one first section (12) is at least in sections of its longitudinal extension direction divided in approximately two equally wide elastic correction strap strips (22), or in which the at least one first section (18) has a slit-like cut (24) in at least one specific position.

3. The elastic correction strap according to claim 1 or 2, in which the at least one first section (12) and/or the at least one third section (18) in each case merge with the correction strap (10).

4. The elastic correction strap according to one of the previous claims, in which the at least one first section (12) and/or the at least one third section (18) are in each case fastened to the elastic correction strap (10) in a material-bonded and/or in a force-locking and/or in another manner.

5. A system for use in the case of toe malpositions of a human foot, which system comprises an elastic correction strap (10) according to one of the claims 1 to 4.

## Revendications

1. Bande de correction élastique (10) pour ou destinée à l'utilisation en cas de malpositions des orteils, présentant :
- au moins une première section (12) qui, en utilisation, est amenée au moins autour d'un orteil (28) d'un pied (32) et forme une boucle (14),
- au moins une deuxième section (16) qui se raccorde à ladite boucle (14) et qui, en utilisation, s'étend en longueur, au moins en partie, de manière médiale le long d'un côté longitudinal d'un pied jusqu'au talon (34),
- au moins une troisième section (18) qui, en utilisation, est amenée autour du talon (34) du pied (32),
**caractérisée en ce que** ladite au moins une troisième section (18) est agencée à l'opposé de ladite deuxième section (16) de la bande de correction élastique (10) qui se raccorde à la boucle (14),
et que ladite au moins une troisième section (18) est, en utilisation, amenée ou peut être amenée orientée de manière oblique en passant sur la plante du pied (32) de sorte que ladite au moins une troisième section (18) forme typiquement un angle compris entre 10° et 170°, de préférence entre 30 et 70° par rapport à ladite au moins une deuxième section (16) qui se raccorde à la boucle (14).

2. Bande de correction élastique selon la revendication 1, dans laquelle ladite au moins une première section (12) est divisée, au moins par sections, dans sa direction d'extension longitudinale en environ deux rubans de bande de correction (22) de même largeur, ou dans laquelle ladite au moins une première section (18) possède un évidement (24) en forme de fente à au moins une position déterminée.

3. Bande de correction élastique selon la revendication 1 ou 2, dans laquelle ladite au moins une première section (12) et/ou ladite au moins une troisième section (18) se confondent respectivement avec la bande de correction (10).

4. Bande de correction élastique selon l'une quelconque des revendications précédentes, dans laquelle ladite au moins une première section (12) et/ou ladite au moins une troisième section (18) est fixée respectivement à la bande de correction élastique (10) par liaison de matériau et/ou par solidarisation et/ou de toute autre manière.

5. Système pour ou destiné à l'utilisation en cas de malformation des orteils d'un pied humain, qui comprend une bande de correction élastique (10) selon les revendications 1 à 4.
